# EUROPEAN PATENT SPECIFICATION

(11) **EP 1 222 929 B1**
(45) Date of publication and mention of the grant of the patent: **19.05.2010**
(21) Application number: 02447005.6
(22) Date of filing: 11.01.2002
(51) Int. Cl.: A61K 39/395, A61P 7/02

(54) **Method and pharmaceutical composition for preventing and/or treating systemic inflammatory response syndrome**
Verfahren und pharmazeutische Zusammensetzung zur Vorbeugung und/oder Behandlung des Syndroms der systemischen entzündlichen Reaktion
Procédé et composition pharmaceutique pour la prévention et/ou le traitement du syndrome de réponse inflammatoire systémique

(30) Priority: 11.01.2001 US 261405 P
(43) Date of publication of application: 17.07.2002
(73) Proprietor: D. Collen Research Foundation vzw, 3000 Leuven (BE)
(72) Inventor: Jacquemin, Marc G., 5330 Sart-Bernard (BE); Saint-Remy, Jean-Marie R., 1390 Grez-Doiceau (BE)
(74) Representative: Bird, William Edward

(56) References cited:
- WO-A-01/04269
- WO-A-97/26010
- JACQUEMIN M ET AL: "A human antibody directed to the factor VIII C1 domain inhibits factor VIII cofactor activity and binding to von Willebrand factor" BLOOD, W.B.SAUNDERS COMPAGNY, ORLANDO, FL, US, vol. 95, no. 1, 1 January 2000 (2000-01-01), pages 156-163, XP002150704 ISSN: 0006-4971
- JACQUEMIN M G ET AL: "MECHANISM AND KINETICS OF FACTOR VIII INACTIVATION: STUDY WITH AN IGG4 MONOCLONAL ANTIBODY DERIVED FROM A HEMOPHILIA A PATIENT WITH INHIBITOR" BLOOD, W.B. SAUNDERS, PHILADELPHIA, VA, US, vol. 92, no. 2, 1998, pages 496-506, XP000906844 ISSN: 0006-4971
- SAINT-REMY JEAN-MARIE R: "B- and T-cell tolerance: From basic concepts to clinical practice." HAEMATOLOGICA, vol. 85, no. 10 Suppl., October 2000 (2000-10), pages 93-96, XP008006242 ISSN: 0390-6078

### SEQUENCE LISTING

<110> D. Collen Research Foundation vzw
   Jacquemin, Marc G
   Saint-Remy, Jean-Marie R
<120> Method and pharmaceutical composition for preventing and
   /or
   treating systemic inflammatory response syndrome
<130> C1968
<150> US 60/261,405
   <151> 2001-01-11
<160> 8
<170> PatentIn version 3.1
<210> 1
   <211> 450
   <212> DNA
   <213> Homo sapiens
<220>
   <221> V_region
   <222> (1)..(450)
   <223>
<220>
   <221> CDS
   <222> (1)..(450)
   <223>
<220>
   <221> misc_feature
   <222> (130)..(159)
   <223> complementary determining region number one
<220>
   <221> misc_feature
   <222> (202)..(258)
   <223> complementary determining region number two
<220>
   <221> misc_feature
   <222> (343)..(375)
   <223> complementary determining region number three
<400> 1
   atg gac tgg acc tgg agg atc ctc ttc ttg gtg gca gca gct aca ggc 48
   Met Asp Trp Thr Trp Arg Ile Leu Phe Leu Val Ala Ala Ala Thr Gly
   1 5 10 15
   acc cac gcc cag gtc caa ctg gta cag tct ggg gct gag gtg aag aag 96
   Thr His Ala Gln Val Gln Leu Val Gln Ser Gly Ala Glu Val Lys Lys
   20 25 30
   cct ggg gcc tca gtg aag gtc tcc tgc aag gtt tcc gga tac acc ctc 144
   Pro Gly Ala Ser Val Lys Val Ser Cys Lys Val Ser Gly Tyr Thr Leu
   35 40 45
   act gaa tta ccc gtg cac tgg gtc gga cag gct cct gga aaa ggg ctt 192
   Thr Glu Leu Pro Val His Trp Val Gly Gln Ala Pro Gly Lys Gly Leu
   50 55 60
   gag tgg gtg gga agt ttt gat cct gaa agt gga gaa tca atc tac gca 240
   Glu Trp Val Gly Ser Phe Asp Pro Glu Ser Gly Glu Ser Ile Tyr Ala
   65 70 75 80
   cgg gag ttc cag ggc agc gtc acc atg acc gcg gac aca tct aca gac
    288
   Arg Glu Phe Gln Gly Ser Val Thr Met Thr Ala Asp Thr Ser Thr Asp
                   85                  90                  95
   ata gcc tac atg gag ctg agc agc ctg aga tct gac gac acg gcc gtg
    336
   Ile Ala Tyr Met Glu Leu Ser Ser Leu Arg Ser Asp Asp Thr Ala Val
               100                 105                 110
   tat tac tgt gca gtc cct gac cct gat gct ttt gat atc tgg ggc caa
    384
   Tyr Tyr Cys Ala Val Pro Asp Pro Asp Ala Phe Asp Ile Trp Gly Gln
           115                 120                 125
   ggg aca atg gtc acc gtc tct tca gcc tcc acc aag ggc cca tcg gtc
    432
   Gly Thr Met Val Thr Val Ser Ser Ala Ser Thr Lys Gly Pro Ser Val
       130                 135                 140
   ttc ccc ctg gga tcc cgt
    450
   Phe Pro Leu Gly Ser Arg
   145                 150
<210> 2
   <211> 150
   <212> PRT
   <213> Homo sapiens
<220>
   <221> misc_feature
   <222> (130)..(159)
   <223> complementary determining region number one
<220>
   <221> misc_feature
   <222> (202)..(258)
   <223> complementary determining region number two
<220>
   <221> misc_feature
   <222> (343)..(375)
   <223> complementary determining region number three
<400> 2
   Met Asp Trp Thr Trp Arg Ile Leu Phe Leu Val Ala Ala Ala Thr Gly
   1               5                   10                  15
   Thr His Ala Gln Val Gln Leu Val Gln Ser Gly Ala Glu Val Lys Lys
               20                  25                  30
   Pro Gly Ala Ser Val Lys Val Ser Cys Lys Val Ser Gly Tyr Thr Leu
           35                  40                  45
   Thr Glu Leu Pro Val His Trp Val Gly Gln Ala Pro Gly Lys Gly Leu
       50                  55                  60
   Glu Trp Val Gly Ser Phe Asp Pro Glu Ser Gly Glu Ser Ile Tyr Ala
   65                  70                  75                  80
   Arg Glu Phe Gln Gly Ser Val Thr Met Thr Ala Asp Thr Ser Thr Asp
   85 90 95
   Ile Ala Tyr Met Glu Leu Ser Ser Leu Arg Ser Asp Asp Thr Ala Val
   100 105 110
   Tyr Tyr Cys Ala Val Pro Asp Pro Asp Ala Phe Asp Ile Trp Gly Gln
   115 120 125
   Gly Thr Met Val Thr Val Ser Ser Ala Ser Thr Lys Gly Pro Ser Val
   130 135 140
   Phe Pro Leu Gly Ser Arg
   145 150
<210> 3
   <211> 426
   <212> DNA
   <213> Homo sapiens
<220>
   <221> V_region
   <222> (1)..(426)
   <223>
<220>
   <221> CDS
   <222> (1)..(426)
   <223>
<220>
   <221> misc_feature
   <222> (127)..(162)
   <223> complementary determining region number one
<220>
   <221> misc_feature
   <222> (205)..(225)
   <223> complementary determining region number two
<220>
   <221> misc_feature
   <222> (325)..(351)
   <223> complementary determining region number three
<400> 3
   atg gaa acc cca gct cag ctt ctc ttc ctc ctg cta ctc tgg ctc cca      48
   Met Glu Thr Pro Ala Gln Leu Leu Phe Leu Leu Leu Leu Trp Leu Pro
   1               5                   10                  15
   gat acc acc gga gaa att gcg ttg acg cag tct cca ggc acc ctg tct      96
   Asp Thr Thr Gly Glu Ile Ala Leu Thr Gln Ser Pro Gly Thr Leu Ser
               20                  25                  30
   ttg tct cca ggg gaa aga gcc acc ctc tcc tgc agg gcc agt cag agt     144
   Leu Ser Pro Gly Glu Arg Ala Thr Leu Ser Cys Arg Ala Ser Gln Ser
           35                  40                  45
   ttt agc agc agc tac tta gcc tgg tat cag cag aaa cct ggc cag gct     192
   Phe Ser Ser Ser Tyr Leu Ala Trp Tyr Gln Gln Lys Pro Gly Gln Ala
       50                  55                  60
   ccc agg ctc ctc atc tat ggt gca tcc acc agg gcc act ggc atc cca 240
   Pro Arg Leu Leu Ile Tyr Gly Ala Ser Thr Arg Ala Thr Gly Ile Pro
   65 70 75 80
   gac agg ttc agt ggc agt ggg tct ggg aca gac ttc act ctc acc atc 288
   Asp Arg Phe Ser Gly Ser Gly Ser Gly Thr Asp Phe Thr Leu Thr Ile
   85 90 95
   agc aga ctg gag cct gaa gat ttt gca gtg tat tac tgt cag aag tat 336
   Ser Arg Leu Glu Pro Glu Asp Phe Ala Val Tyr Tyr Cys Gln Lys Tyr
   100 105 110
   ggt acg tca gcg atc acc ttc ggg caa ggg aca cga ctg gag att aaa 384
   Gly Thr Ser Ala Ile Thr Phe Gly Gln Gly Thr Arg Leu Glu Ile Lys
   115 120 125
   gga act gtg gct gca cca tct gtc ttc atc ttc ccg cca tct 426
   Gly Thr Val Ala Ala Pro Ser Val Phe Ile Phe Pro Pro Ser
   130 135 140
<210> 4
   <211> 142
   <212> PRT
   <213> Homo sapiens
<220>
   <221> misc_feature
   <222> (127)..(162)
   <223> complementary determining region number one
<220>
   <221> misc_feature
   <222> (205)..(225)
   <223> complementary determining region number two
<220>
   <221> misc_feature
   <222> (325)..(351)
   <223> complementary determining region number three
<400> 4
   Met Glu Thr Pro Ala Gln Leu Leu Phe Leu Leu Leu Leu Trp Leu Pro
   1               5                   10                  15
   Asp Thr Thr Gly Glu Ile Ala Leu Thr Gln Ser Pro Gly Thr Leu Ser
               20                  25                  30
   Leu Ser Pro Gly Glu Arg Ala Thr Leu Ser Cys Arg Ala Ser Gln Ser
           35                  40                  45
   Phe Ser Ser Ser Tyr Leu Ala Trp Tyr Gln Gln Lys Pro Gly Gln Ala
       50                  55                  60
   Pro Arg Leu Leu Ile Tyr Gly Ala Ser Thr Arg Ala Thr Gly Ile Pro
   65                  70                  75                  80
   Asp Arg Phe Ser Gly Ser Gly Ser Gly Thr Asp Phe Thr Leu Thr Ile
                   85                  90                  95
   Ser Arg Leu Glu Pro Glu Asp Phe Ala Val Tyr Tyr Cys Gln Lys Tyr
               100                 105                 110
   Gly Thr Ser Ala Ile Thr Phe Gly Gln Gly Thr Arg Leu Glu Ile Lys
           115                 120                 125
   Gly Thr Val Ala Ala Pro Ser Val Phe Ile Phe Pro Pro Ser
       130                 135                 140
<210> 5
   <211> 468
   <212> DNA
   <213> Homo sapiens
<220>
   <221> V_region
   <222> (1)..(468)
   <223>
<220>
   <221> CDS
   <222> (1)..(468)
   <223>
<220>
   <221> misc_feature
   <222> (124)..(192)
   <223> complementary determining region number one
<220>
   <221> misc_feature
   <222> (232)..(285)
   <223> complementary determining region number two
<220>
   <221> misc_feature
   <222> (282)..(435)
   <223> complementary determining region number three
<400> 5
   atg gac tgg acc tgg agg atc ctc ttc ttg gtg gca gca gcc aca gga
     48
   Met Asp Trp Thr Trp Arg Ile Leu Phe Leu Val Ala Ala Ala Thr Gly
   1               5                   10                  15
   gcc cac tcc cag gtg caa ctg gtg caa tct ggg gct gag gtg aag aag
     96
   Ala His Ser Gln Val Gln Leu Val Gln Ser Gly Ala Glu Val Lys Lys
               20                  25                  30
   cct ggg gcc tca gtg aag gtc tcc tgc aag acc tct gga tac aac ttc
    144
   Pro Gly Ala Ser Val Lys Val Ser Cys Lys Thr Ser Gly Tyr Asn Phe
           35                  40                  45
   acc ggc tac tct gct tct gga cat atc ttc acc gcc tac tct gtg cac
    192
   Thr Gly Tyr Ser Ala Ser Gly His Ile Phe Thr Ala Tyr Ser Val His
       50                  55                  60
   tgg gtg cga cag gcc cct gga caa ggg ctt gag tgg atg gga agg atc
    240
   Trp Val Arg Gln Ala Pro Gly Gln Gly Leu Glu Trp Met Gly Arg Ile
   65                  70                  75                  80
   aac cct aac agt ggt gcc aca gac tat gca cat aaa ttt cag ggc agg
    288
   Asn Pro Asn Ser Gly Ala Thr Asp Tyr Ala His Lys Phe Gln Gly Arg
                   85                  90                  95
   gtc acc atg tcc agg gac acg tcc atc agc aca gcc tac atg gaa ctg
    336
   Val Thr Met Ser Arg Asp Thr Ser Ile Ser Thr Ala Tyr Met Glu Leu
               100                 105                 110
   agc agg ctg aca tct gac gac acg gcc atg tat tac tgt gcg aga gcc
    384
   Ser Arg Leu Thr Ser Asp Asp Thr Ala Met Tyr Tyr Cys Ala Arg Ala
           115                 120                 125
   gac aac tat ttc gat att gtg act ggc tat act tct cat tac ttt gac
    432
   Asp Asn Tyr Phe Asp Ile Val Thr Gly Tyr Thr Ser His Tyr Phe Asp
       130                 135                 140
   tac tgg ggc cgg gga acc ctg gtc acc gtc tcc tca
    468
   Tyr Trp Gly Arg Gly Thr Leu Val Thr Val Ser Ser
   145                 150                 155
<210> 6
   <211> 156
   <212> PRT
   <213> Homo sapiens
<220>
   <221> misc_feature
   <222> (124)..(192)
   <223> complementary determining region number one
<220>
   <221> misc_feature
   <222> (232)..(285)
   <223> complementary determining region number two
<220>
   <221> misc_feature
   <222> (282)..(435)
   <223> complementary determining region number three
<400> 6
   Met Asp Trp Thr Trp Arg Ile Leu Phe Leu Val Ala Ala Ala Thr Gly
   1               5                   10                  15
   Ala His Ser Gln Val Gln Leu Val Gln Ser Gly Ala Glu Val Lys Lys
               20                  25                  30
   Pro Gly Ala Ser Val Lys Val Ser Cys Lys Thr Ser Gly Tyr Asn Phe
           35                  40                  45
   Thr Gly Tyr Ser Ala Ser Gly His Ile Phe Thr Ala Tyr Ser Val His
       50                  55                  60
   Trp Val Arg Gln Ala Pro Gly Gln Gly Leu Glu Trp Met Gly Arg Ile
   65                  70                  75                  80
   Asn Pro Asn Ser Gly Ala Thr Asp Tyr Ala His Lys Phe Gln Gly Arg
                   85                  90                  95
   Val Thr Met Ser Arg Asp Thr Ser Ile Ser Thr Ala Tyr Met Glu Leu
               100                 105                 110
   Ser Arg Leu Thr Ser Asp Asp Thr Ala Met Tyr Tyr Cys Ala Arg Ala
           115                 120                 125
   Asp Asn Tyr Phe Asp Ile Val Thr Gly Tyr Thr Ser His Tyr Phe Asp
       130                 135                 140
   Tyr Trp Gly Arg Gly Thr Leu Val Thr Val Ser Ser
   145                 150                 155
<210> 7
   <211> 429
   <212> DNA
   <213> Homo sapiens
<220>
   <221> V_region
   <222> (1)..(429)
   <223>
<220>
   <221> CDS
   <222> (1) .. (429)
   <223>
<220>
   <221> misc_feature
   <222> (127)..(162)
   <223> complementary determining region number one
<220>
   <221> misc_feature
   <222> (205)..(225)
   <223> complementary determining region number two
<220>
   <221> misc_feature
   <222> (325)..(354)
   <223> complementary determining region number three
<400> 7
   atg gaa acc cca gct cag ctt ctc ttc ctc ctg cta ctc tgg ctc cca      48
   Met Glu Thr Pro Ala Gln Leu Leu Phe Leu Leu Leu Leu Trp Leu Pro
   1               5                   10                  15
   gat acc acc gga gaa att gtg ttg acg cag ttc cca ggc acc ctg tct      96
   Asp Thr Thr Gly Glu Ile Val Leu Thr Gln Phe Pro Gly Thr Leu Ser
               20                  25                  30
   ttg tct cca ggg gaa aga gcc acc ctc tcc tgc agg gcc agt cag agt     144
   Leu Ser Pro Gly Glu Arg Ala Thr Leu Ser Cys Arg Ala Ser Gln Ser
           35                  40                  45
   gtt gcc agc gcc tac tta gcc tgg tac cag caa aaa cct ggc cag gct     192
   Val Ala Ser Ala Tyr Leu Ala Trp Tyr Gln Gln Lys Pro Gly Gln Ala
       50                  55                  60
   ccc agg ctc ctc atc tat ggt gca tcc agt agg gcc acc gac atc cca
    240
   Pro Arg Leu Leu Ile Tyr Gly Ala Ser Ser Arg Ala Thr Asp Ile Pro
   65                  70                  75                  80
   cac agg ttc agt ggc agt ggg tct ggg aca gac ttc act ctc acc atc
    288
   His Arg Phe Ser Gly Ser Gly Ser Gly Thr Asp Phe Thr Leu Thr Ile
                   85                  90                  95
   agc aga ctg gag cct gaa gat ttt gca gtg tac tac tgt cag caa tat
    336
   Ser Arg Leu Glu Pro Glu Asp Phe Ala Val Tyr Tyr Cys Gln Gln Tyr
               100                 105                 110
   ggt acc tca gcc tta ctc act ttc ggc gga ggg acc aag gtg gag atc
    384
   Gly Thr Ser Ala Leu Leu Thr Phe Gly Gly Gly Thr Lys Val Glu Ile
           115                 120                 125
   aaa cga act gtg gct gca cca tct gtc ttc atc ttc ccg cca tct
    429
   Lys Arg Thr Val Ala Ala Pro Ser Val Phe Ile Phe Pro Pro Ser
       130                 135                 140
<210> 8
   <211> 143
   <212> PRT
   <213> Homo sapiens
<220>
   <221> misc_feature
   <222> (127)..(162)
   <223> complementary determining region number one
<220>
   <221> misc_feature
   <222> (205)..(225)
   <223> complementary determining region number two
<220>
   <221> misc_feature
   <222> (325)..(354)
   <223> complementary determining region number three
<400> 8
   Met Glu Thr Pro Ala Gln Leu Leu Phe Leu Leu Leu Leu Trp Leu Pro
   1               5                   10                  15
   Asp Thr Thr Gly Glu Ile Val Leu Thr Gln Phe Pro Gly Thr Leu Ser
               20                  25                  30
   Leu Ser Pro Gly Glu Arg Ala Thr Leu Ser Cys Arg Ala Ser Gln Ser
           35                  40                  45
   Val Ala Ser Ala Tyr Leu Ala Trp Tyr Gln Gln Lys Pro Gly Gln Ala
       50                  55                  60
   Pro Arg Leu Leu Ile Tyr Gly Ala Ser Ser Arg Ala Thr Asp Ile Pro
   65                  70                  75                  80
   His Arg Phe Ser Gly Ser Gly Ser Gly Thr Asp Phe Thr Leu Thr Ile
   85 90 95
   Ser Arg Leu Glu Pro Glu Asp Phe Ala Val Tyr Tyr Cys Gln Gln Tyr
   100 105 110
   Gly Thr Ser Ala Leu Leu Thr Phe Gly Gly Gly Thr Lys Val Glu Ile
   115 120 125
   Lys Arg Thr Val Ala Ala Pro Ser Val Phe Ile Phe Pro Pro Ser
   130 135 140

## Description

The present invention describes the prevention and/or treatment of systemic inflammatory response syndrome associated to infection in mammals. The invention further describes pharmaceutical compositions for the therapeutic treatment of sepsis and/or septic shock, as well as septicemia.

### BACKGROUND OF THE INVENTION

Systemic inflammation is the possible endpoint of a number of clinical conditions including pancreatitis, ischemia, multiple trauma and tissue injury, haemorrhagic shock, immune-mediated organ injury and infection. Since quite comparable pathological changes are observed in systemic inflammation independently of the initial cause, the term "systemic inflammatory response syndrome" (hereinafter referred as SIRS) has been commonly quoted to account for such changes and is therefore used in the present application in accordance with the recommendations of the American College of Chest Physicians as formulated by R.C.Bone et al. in Chest (1992) 101:1644-55.

Sepsis represents a SIRS associated to infection. Shock, whether of septic origin or not, is characterized namely by (a) hypotension persisting despite adequate fluid resuscitation and (b) abnormalities related to hypoperfusion or organ dysfunction.

SIRS, and sepsis in particular, is a quite frequent disorder. Recent estimates have indicated that about 400,000 cases of sepsis are diagnosed every year in the United States of America, with about 50% of them evolving to septic shock. The latter is associated with a mortality rate of about 50%. Furthermore, the frequency of sepsis and septic shocks is increasing despite the use of efficient antibiotic therapy and resuscitation measures based on recent understanding of the patho-physiology of sepsis and its complications. This frequency increase is thought to be related to an increased number of immuno-compromised patients, an expanding population of elderly patients and a significant resistance to some anti-microbial therapies.

According to Bone et al. in Chest (1997) 112:235-43, the early systemic inflammation, which is an integral part of SIRS, is normally compensated by a later anti-inflammatory response. An equilibrium between pro-inflammatory and anti-inflammatory events leads to a favorable clinical outcome. However, the balance between these two antagonistic events is fragile and easily disrupted, since many interacting mediators are involved.

In recent years much knowledge has accumulated about the pathophysiology of SIRS and the development of related septic shock, the paradigm of which is due to infection with gram (-) microorganisms. Thus endotoxins liberated by such microorganisms activate a number of host cells and in particular monocytes and macrophages. This results in the production of pro-inflammatory cytokines including tumor necrosis factor (hereinafter referred as TNF), interleukins 1, 6 and 8 (hereinafter referred IL-1, IL-6 and IL-8). Enzymes produced by the same cells lead to activation of the coagulation, complement and bradykinin systems. Superoxide radicals and metabolites of arachidonic acid, including platelet activating factor (hereinafter referred as PAF) are produced according to Parillo et al. in Ann. Intern. Med. (1990) 113:227-242.

Activation of the coagulation system together with adhesion of polymorphonuclear cells and platelets to the vascular endothelium is essential in the pathogenesis of SIRS since it results in the formation of thrombus in the microvasculature. Thrombi impair organ perfusion, which ends up with organ dysfunction or failure. At the intestinal level, this produces an increased absorption of endotoxins from the bacterial flora, which further boosts the release of pro-inflammatory cytokines and organ dysfunction. The disseminated intravascular coagulation (hereinafter referred as DIC) is therefore an essential component of the pathophysiology of sepsis and septic shock.

A compensatory anti-inflammatory response emerges later, from which the clinical outcome of sepsis will depend. Too weak or too strong responses can aggravate the clinical situation by failure to control DIC or by leading to uncontrolled bleeding and immune suppression with increased susceptibility to further infection. The individual factors intervening in this anti-inflammatory phase are not all identified with certainty but a number of molecules have been clearly implicated. Thus the levels of anti-thrombin (hereinafter referred as AT, a plasma serine protease inhibitor with a normal plasma concentration of 3 µmol/L), activated protein C (APC) and tissue factor pathway inhibitor (hereinafter referred as TFPI, another endothelial-bound protein), three potent inhibitors acting at various steps in the coagulation cascade, are severely reduced during sepsis and their plasma concentrations are inversely correlated with bad clinical prognosis according to E.F. Mammen in Intensive Care Med. (1998) 24:649-50. Such reduced levels are due both to increased consumption and reduced synthesis, namely by the liver.

The coagulation system is currently divided in an initiation phase, an amplification phase and an effective phase. Initiation occurs by activation of factor VII on tissue factor (TF) or by the contact factor XII. This results in the generation of small amounts of thrombin, which activates an amplification loop leading to more thrombin formation. Two co-factors in this amplification loop, factor V and factor VIII, are activated, their function being to increase by several logs of magnitude the cleavage of prothrombin and factor X respectively. The effective phase of the coagulation cascade eventually leads to the formation of fibrin and clot retraction. Thrombin therefore occupies a central role in the development of DIC associated with sepsis or SIRS of other origin. This discovery has led to therapeutic attempts to reduce thrombin formation. In humans, studies comparing antithrombin with a synthetic protease inhibitor (Maki et al. in Gynecol. Obstet. Invest. (1987) 23:230-240) or heparin (Blauhut et al. in Thromb. Res. (1985) 39:81-89) documented a significant attenuation of disseminated intravascular coagulation after antithrombin treatment, but neither included a placebo control group. According to Fourrier et al. in *DIC*, Excerpta Medica, Amsterdam (1993) 221-226, a placebo-controlled, double blind trial in patients with septic shock and DIC treatment with a plasma concentrate of antithrombin achieved significantly earlier correction of DIC but failed to decrease mortality in a significant manner. More recently, plasma-derived or recombinant AT have been tested in the control of sepsis, namely according to Eisele et al. in Intensive Care Med. (1998) 24:663-72. However all these embodiments encountered serious problems. Natural plasma antithrombin is a relatively poor inhibitor of thrombin (it achieves full inhibition of thrombin, but only at very high concentrations), but its inhibitory effect is increased 10,000 fold in the presence of heparin. High concentrations of antithrombin are necessary to prevent shock in animal models of sepsis, according to Büller et al. in Am. J. Med. (1989) 87:44-48 and Vinazzer in Clin. Appl. Thromb/Hemost. (1995) 1:62-65. Because of the moderate survival time of antithrombin in the circulation (a half-life of about 3 days was reported by Schwartz et al. in Am. J. Med. (1989) 87:53-60 and Menache et al. in Blood (1990) 75:33-39) and its consumption in SIRS, its activity should be monitored regularly. Theoretically, combined antithrombin and heparin therapy should be more effective than antithrombin alone in the management of shock, but unfortunately this form of treatment did not improve the outcome in shocked patients and was associated with an increased risk of bleeding.

WO9726010 describes a method for inhibiting thrombosis based on the administration of an anti-factor VIII monoclonal antibody having self-limiting neutralising activity and pharmaceutical compositions comprising this type of anti factor VIII monoclonal antibody.

Jacquemin et al. (2000) Blood 95, 156-163 analysed at the clonal level the immune response of a mild/moderate haemophilia patient with an inhibitor to wild-type but not self-fVIIIl and an Arg2150His substitution in the C1 domain. Immortalization of the patient B lymphocytes provided a cell line producing an anti-fVIII IgG4kappa antibody, LE2E9, that inhibited factor VIII cofactor activity and prevented factor VIII binding to von Willebrand factor. Epitope mapping with recombinant factor VIII fragments indicated that LE2E9 recognised the factor VIII C1 domain, but not the Arg2150His-substituted C1 domain. LE2E9 did not inhibit Arg2150His factor VIII activity. This identified C1 as a novel target for factor VIII inhibitors and demonstrates that Arg2150His substitution alters a B-cell epitope in the C1 domain.

PCT application WO0104269 has been filed on July 13, 2000, claiming a priority of July 14, 1999 and was published on January 18, 2001, and is relevant for the novelty of the present application under Art. 54(3) EPC. WO0104269 discloses pharmaceutical compositions comprising as active ingredient the human monoclonal antibody obtainable from the cell line named KRIX1, which is the cell line deposited under the accession number 5089CB. This document further discloses a method of treatment and/or prevention of a thrombotic pathological condition, inter alia arterial or venous thrombosis, based on the administration of the human monoclonal antibody obtainable from the KRIX1 cell line.
There remains a strong need in the art for a safer and more effective therapy and/or prevention of a systemic inflammatory response syndrome and related shock and, more specifically, for the treatment of blood circulation disorders following a systemic infection with gram (-) microorganisms.

### SUMMARY OF THE INVENTION

According to the present invention, factor VIII is however a more appropriate target in order to maintain the rate of thrombin formation under control in mammals. This is namely because factor VIII acts as a cofactor in the formation of the tenase complex, i.e. the enzyme which activates factor X, which in turn converts pro-thrombin into thrombin in the presence of factor V. As a co-factor, factor VIII is not absolutely required for the generation of thrombin and some coagulation still occurs in its absence. On the other hand, according to M. Hultin in J. Clin. Invest. (1981) 69:950-8, factor VIII increases by 100,000-fold the rate of tenase formation. Therefore the present invention is based on controlling the activity of factor VIII, this being an efficient way to slow down the formation of thrombin. More broadly, this invention is based on partially inhibiting the formation of thrombin (i.e. decreasing or reducing the rate of thrombin formation) rather than fully inhibiting said formation as is achieved by antithrombin or a heparin-antithrombin combination.

Herein disclosed are partial inhibitors of factor VIII for preventing and/or treating a systemic inflammatory response syndrome (SIRS) in mammals by partially inhibiting the formation of thrombin, for instance by administering a partial inhibitor of factor VIII to the mammal in need thereof. This invention also provides a method for preventing and/or treating sepsis and/or septic shock and/or treating thrombus formation in the micro-vasculature in mammals. Herein disclosed is furthermore a partial inhibitor of factor VIII for restoring the plasma level of anti-thrombin and/or activated protein C and/or tissue factor pathway inhibitor in mammals by administering to the mammal in need thereof. Herein described are pharmaceutical compositions for use in the above-referred prevention and/or treatment, comprising as an active ingredient a partial inhibitor of factor VIII, in admixture with a pharmaceutically acceptable carrier.

The invention also relates to the use of a partial inhibitor of factor VIII for the manufacture of a medicine for the prevention and/or treatment of systemic inflammatory response syndrome associated to infection and/or sepsis and/or septic shock and/or septicemia in mammals, wherein the partial inhibitor is a monoclonal antibody or a fragment thereof binding to a site of factor VIII or of a complex involving factor VIII, and having the capacity of inactivating factor VIII or a complex involving factor VIII by at least about 25 % and at most 95%, as determined by a chromogenic assay, when the monoclonal antibody or fragment is in a molar excess with respect to factor VIII.

According to one embodiment, the partial inhibitor is a monoclonal antibody or fragment which recognizes an epitope located in the C1 domain of factor VIII

According to another embodiment, the partial inhibitor is a human monoclonal antibody obtainable from the cell line named KRIX-1 deposited with the Belgian Coordinated Collections of Micro-organisms under accession number LMBP 5089CB, or an antibody having at least 80% sequence homology therewith within the CDR regions, or an antigen-binding fragment thereof.

According to an alternative embodiment, the partial inhibitor is a monoclonal antibody having a reactivity identical to that of human monoclonal antibodies obtained from the cell line KRIX-1 deposited with the Belgian Coordinated Collections of Micro-organisms under accession number LMBP 5089CB, or an antigen-binding fragment thereof.

According to a further particular embodiment the fragment of the antibody is an Fab, Fab' or F(ab')₂ fragment, a soluble or membrane-anchored single-chain variable fragment, or a single variable domain or a combination thereof.

In an alternative embodiment the medicine further comprises a therapeutically effective amount of an anti-thrombotic agent, such as heparin.

### BRIEF DESCRIPTION OF THE DRAWINGS

Figure 1 shows producing human monoclonal antibodies derived from a hemophilia A patient, expressed in the form of IgG antibody binding to factor VIII in ELISA.
Figure 2 shows inhibition of factor VIII activity by the monoclonal antibody BO2C11.
Figure 3 shows inhibition of factor VIII activity by the monoclonal antibody produced from the cell line KRIX 1.
Figure 4 shows inhibition of binding of activated factor VIII on phosphatidyl-L-serine by the monoclonal antibody produced from the cell line KRIX 1.
Figure 5 shows the influence of certain polyclonal antibodies on the dissociation of activated factor VIII from von Willebrand factor.
Figure 6 shows partial inhibition of thrombin by the monoclonal antibody produced from the cell line KRIX 1.
Figure 7 shows plasma concentrations of plasminogen activation inhibitor type-I in wild-type C57B1/6 mice and in factor VIII knock-out mice after injection of lipopolysaccharide.
Figure 8 shows plasma concentrations of α₂-antiplasmin in wild-type C57B1/6 mice and in factor VIII knock-out mice after injection of lipopolysaccharide.
Figure 9 shows plasma concentrations of fibrinogen in wild-type C57B1/6 mice and in factor VIII knock-out mice after injection of lipopolysaccharide.

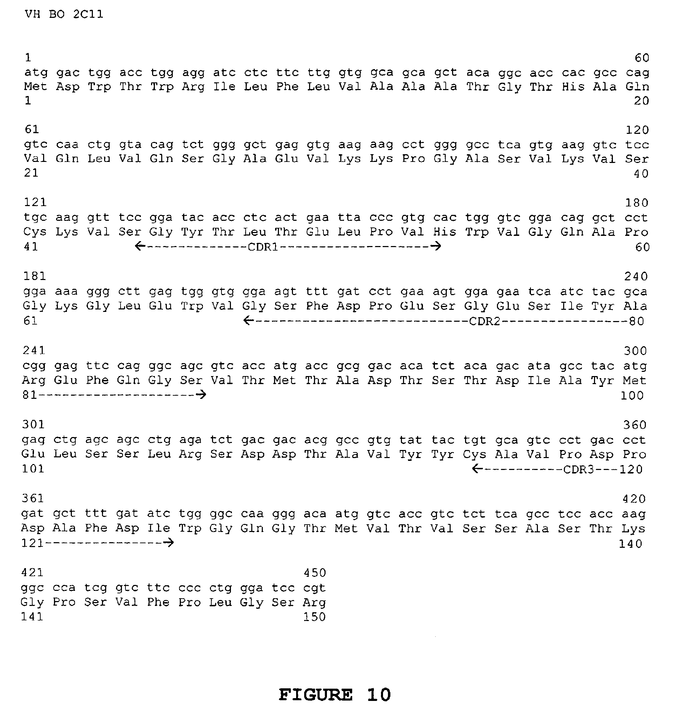

Figure 10 shows the amino-acid sequence SEQ.ID.No.2 (lower lines) and the nucleotide sequence SEQ.ID.No.1 (upper lines) for the variable regions V_{H} of the heavy chains of the human monoclonal antibodies BO2C11. Also shown are the three complementarity determining regions of each chain.

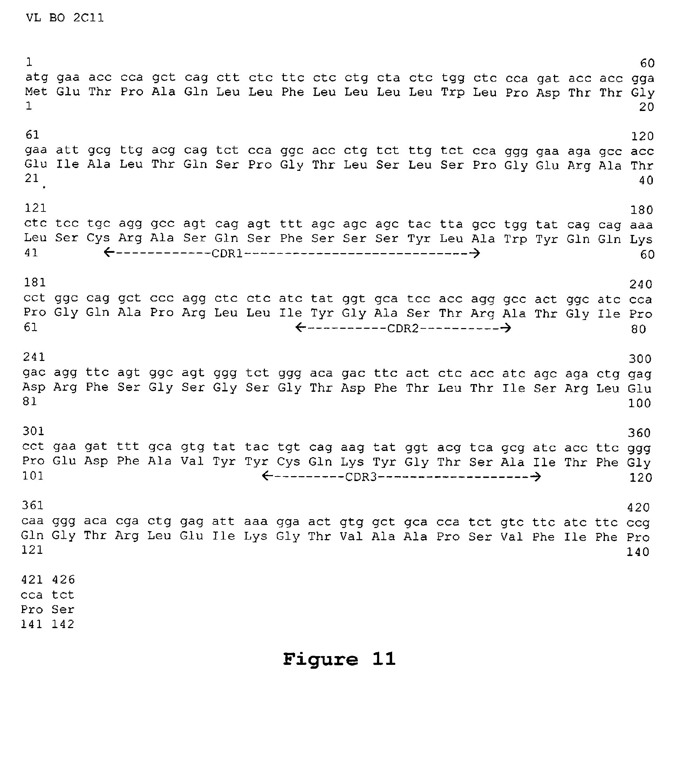

Figure 11 shows the amino-acid sequence SEQ.ID.No.4 (lower lines) and the nucleotide sequence SEQ.ID.No.3 (upper lines) for the variable regions V_{L} of the light chains of the human monoclonal antibodies BO2C11. Also shown are the three complementarity determining regions of each chain.

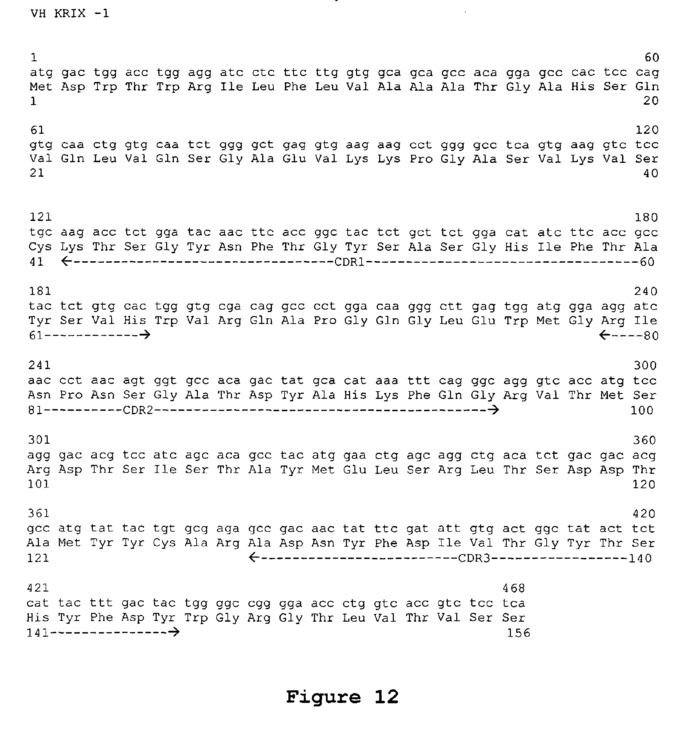

Figure 12 shows the amino-acid sequence SEQ.ID.No.6 (lower lines) and the nucleotide sequence SEQ.ID.No.5 (upper lines) for the variable regions V_{H} of the heavy chains of the KRIX 1 monoclonal antibodies. Also shown are the three complementarity determining regions of each chain.

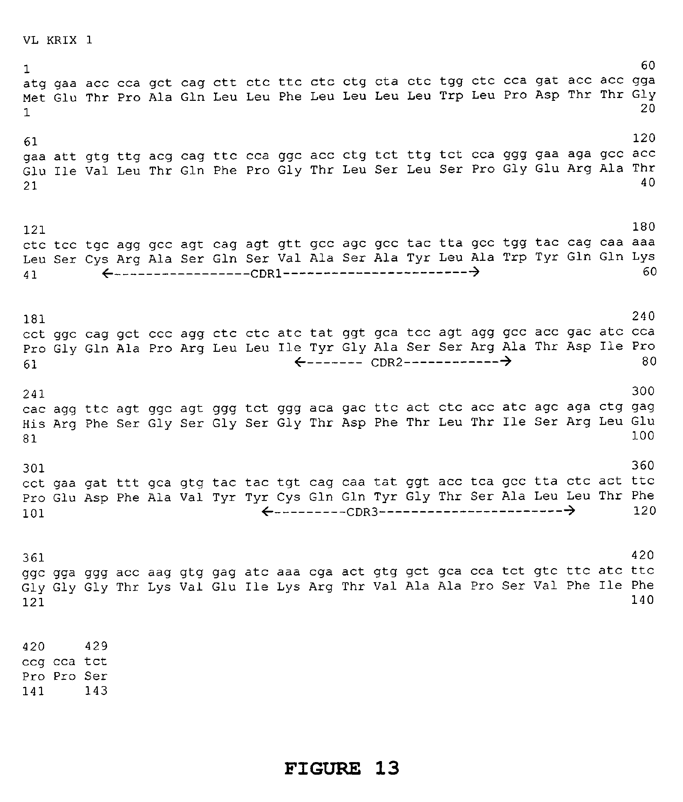

Figure 13 shows the amino-acid sequence SEQ.ID.No.8 (lower lines) and the nucleotide sequence SEQ.ID.No.7 (upper lines) for the variable regions V_{L} of the light chains of the KRIX 1 monoclonal antibodies. Also shown are the three complementarity determining regions of each chain.

### DEFINITIONS

The term "ligand" is used herein with the meaning given at page 127 of The Molecular Biology of the Cell (1983), Garland Publishing Inc., New-York, i.e. a molecule which is able to effectively interact with a protein through the simultaneous formation of a number of weak bonds between them.

The term "antibody" as used herein refers to intact molecules which are capable of binding to the epitope determinant of factor VIII or the relevant domain of factor VIII, The term "fragment" refers to antigen-binding fragments, such as Fab, Fab', F(ab')₂, Fv and the like, of such antibodies.

"Humanized antibody" as used herein refers to antibody molecules in which amino-acids have been replaced in the non-antigen binding regions in order to more closely look like a human antibody. "Reshaped human antibody" or "Human hybrid antibody" refers to a human antibody in which amino acids in the antigen binding regions of a human antibody have been replaced with sequences, e.g. CDR's, or other parts of variable regions which have been derived from the repertoire of human antibodies.

The term "homology" or "homologous" as used herein with reference to ligands (such as antibodies) refers to a molecule which will compete with or inhibit binding of one of the said ligands to the target site. The binding should be specific, i.e. the binding of the alternative molecule should be as specific to the site as the ligand. Where the said ligands include amino-acid sequences, then, homology should include having at least 80%, more preferably 90% and most preferably 95% amino acid sequence identity with the relevant ligand.

### DETAILED DESCRIPTION OF THE INVENTION

Factor VIII is a protein providing coagulant cofactor activity and is one of human clotting factors with a rather high molecular weight (265,000) and a very low normal plasma concentration (0.0007 micromol./litre). With its 2,332 amino-acid residues, factor VIII is one of the longest known polypeptide chains and is synthesized in the liver, the spleen and the placenta. Its gene has been shown to include 186,000 nucleotides.

Factor VIII circulates as inactive plasma protein. Factors V and VIII are homologous proteins sharing a common structural configuration of triplicated A domains and duplicated C domains with structurally divergent B domains connecting the A2 and A3 domains. Factor VIII circulates in a multiplicity of fragmented species in a tightly associated complex with von Willebrand factor at a concentration of 1 nmol/L. Factor VIII activation occurs by a cleavage between the A1 and A2 domains, resulting in the unstable heterotrimeric factor Villa molecule. Factor Villa binds tightly to membranes that contain acidic phospholipids. Factor VIII contains a phospholipid binding site in the C2 domain, between amino-acids 2302 and 2332, according to Arai et al. in J.Clin.Invest. (1989) 83:1978. Within the same factor VIII region, there is also a von Willebrand factor binding site acting in conjunction with amino-acid residues 1645-1689 in the A3 domain according to Shima et al. in Throm.Haemost. (1993) 69:240 and J. Biol. Chem. (1994)269:11601.

Lack or insufficient function of factor VIII results in haemophilia A. Patients with no factor VIII (severe form of the disease) have spontaneous bleedings in joints and soft tissues. In the mild form of the disease, bleedings occur only after trauma.

Inhibiting the function of factor VIII would therefore lead to spontaneous bleeding unless the said function is only partially inhibited. Taking this into account, the present inventors have developed specific anti-factor VIII monoclonal antibodies that inhibit factor VIII co-factor activity only to a limited extent.

Fully human, as well as humanized, monoclonal antibodies with high affinity for factor VIII are provided, which exhibit an only partial inhibition of the function of factor VIII, independently of the dosage used, thereby preventing the risk of overdosing. These antibodies are used in methods of treatment and prevention and in pharmaceutical compositions, as further explained hereinbelow. Antibodies are unique reagents since they offer exquisite specificity for the determinant that they recognize. Anti-factor VIII antibodies bind to factor VIII but not to other, even homologue, proteins. Because of the low plasma concentration of factor VIII, only limited amounts of inhibiting antibodies are required to neutralize all factor VIII molecules. Besides, such antibodies have a half-life of about three weeks in human beings, which advantageously allows to increase intervals between their administration to a mammal in need thereof.

The present invention relates to a general concept of only partially inactivating factor VIII using human or humanized monoclonal antibodies or fragments or homologues thereof, which bind to a site of factor VIII, or a complex involving factor VIII in the manufacture of a medicament for preventing and/or treating systemic inflammatory response syndrome (SIRS) associated to infection and/or sepsis and/or septic shock and/or septicemia in mammals, in particular in humans. These ligands and compositions have the advantageous property that the inactivation of the factor is only partial even when the ligand is in a molar excess. This means that even though the ligand is used in an amount which might be expected to inactivate completely the targeted factor, the inactivation is still incomplete.

Herein disclosed is a particular cell line producing human monoclonal antibodies which are reactive with human factor VIII and more specifically have the capacity of inactivating the co-factor activity of human factor VIII by interfering with proteolytic cleavage site or von Willebrand factor or tenase complex reaction or by inducing a three-dimensional conformational change in factor VIII, in particular by targeting a domain of factor VIII and by recognizing epitopes located in the said domain. One preferred domain is the C1 domain of factor VIII. A site on the C2 domain of factor VIII may also be partially inhibited. Herein disclosed are also ligands other than polyclonal antibodies, in particular monoclonal antibodies, which reduce the release rate of factor VIII from von Willebrand factor. These monoclonal antibodies specifically target factor VIII when bound to von Willebrand factor and hence target an epitope associated with the complex of factor VIII and von Willebrand factor. The present disclosure also provides fragments of any of the above monoclonal antibodies such as Fab, Fab', F(ab')₂, scFv, CDR's, single variable domains as well as homologues and combinations thereof. More particularly, these monoclonal antibodies and fragments may target a domain of factor VIII, in particular the C1 domain of factor VIII. They may also partially inhibit a site on the C2 domain of factor VIII. They may also target an epitope associated with the complex of von Willebrand factor and factor VIII. Herein a new use is provided of ligands, other than polyclonal antibodies, which bind to a first site (e.g. in the C1 domain of factor VIII) remote from a functional second site (e.g. the site in the C2 domain of factor VIII which is responsible for binding phospholipids) in such a way that the function of the second site is only partially impaired even when the ligand is in a molar and therapeutic excess.

The cell line named KRIX 1 producing monoclonal antibodies as used in the present invention was deposited with the BCCM™/LMBP (Belgian Coordinated Collections of Microorganisms/Plasmid Collection Laboratorium voor Moleculaire Biologie, University of Ghent K.L. Ledeganckstraat 35, B-9000 Ghent, BE under accession number LMBP 5089CB on July 1, 1999.

Herein described are cell lines producing human monoclonal antibodies having a reactivity substantially similar to that of the human monoclonal antibodies obtained from the above-mentioned deposited cell line, as well as the human monoclonal antibodies obtainable from these further cell lines.

Herein further described are reshaped human monoclonal antibodies or human hybrid monoclonal antibodies against factor VIII, which bind to and only partially inactivate factor VIII or a complex including factor VIII and which comprise only elements derived from the repertoire of human antibodies. By human hybrid monoclonal antibodies it is meant a hybrid antibody constructed from a human antibody and from variable regions. Conventionally in the art, until now it has only been possible to obtain antibodies against factor VIII derived from animals, e.g. mice, or to construct chimeric antibodies from human antibodies with the variable portions derived from mice antibodies.

The present invention also provides a new use of ligands, other than polyclonal antibodies, having the capacity of only partially inactivating factor VIII or a complex including factor VIII, by binding to a site of the said factor or complex, the said only partial inactivation also taking place when the ligand is in a molar excess with respect to the said factor. The site to which the ligand binds may or may not be directly or substantially involved in a physiological interaction of the said factor or complex. For instance, the ligand may bind to a site which is at a predetermined distance away from a physiologically functional site of the said factor. By partial inactivation, herein we mean an at most 99% inactivation, preferably an at most 95% inactivation, as determined by a suitable test method such as for instance the chromogenic assay available from Coatest™, Kabi Vitrum, Brussels (Belgium) or from Chromogenix AB, Mölndal (Sweden). On the other hand, in order to provide therapeutic usefulness, inactivation of factor VIII should be at least about 25%, preferably at least 60% and more preferably at least about 70%, as determined by the same test method as above. The extent of factor VIII inhibition or inactivation may vary from one patient to another, depending on the acuteness of the systemic inflammatory response syndrome SIRS and of its causality factor.

It will be appreciated that the inhibitors having partial inactivating capacity described herein operate in a different way from the mechanism conventionally ascribed to type II antibodies against factor VIII. One conventional mechanism is that of competition with another factor, e.g. von Willebrand factor. The kinetics of a competition mechanism means that if the one species is at a high concentration compared with the other (e.g. in a molar excess), the inhibition is effectively complete. In contrast, the inhibitors described herein reach a plateau effect in the inactivation of factor VIII, which is substantially independent of the excess of the inhibitor. The other conventional mechanism ascribed to type II antibodies is that of low affinity, in which case an excess will drive the reaction to complete inhibition as well.

The inhibitors useful in this invention may be human monoclonal antibodies obtainable from the deposited cell line KRIX 1, preferably being of class IgG, and which have the capacity of only partially inactivating the co-factor activity of factor VIII. More specifically human monoclonal antibodies are disclosed from such origin which are able to recognize epitopes located in the C1 domain of factor VIII. Although the inventors do not wish to be bound to a single explanation or theory, it is believed that the binding of such human monoclonal antibodies results in partial impairment of the binding of activated factor VIII to phospholipids, a necessary step for cofactor activity expression.

The present invention further provides a new use of monoclonal antibodies having substantially the same characteristics as above disclosed and being produced by on purpose immunization in animals, preferably in mouse, for instance by injecting human factor VIII in mice and then fusing the spleen lymphocytes with a mouse myeloma cell line, followed by identifying and cloning the cell cultures producing anti-factor VIII antibodies. The monoclonal antibodies produced in animals are then humanized, for instance by associating the binding complementarity determining region ("CDR") from the non-human monoclonal antibody with human framework regions - in particular the constant C region of human gene - such as disclosed by Jones et al. in Nature (1986) 321:522 or Riechmann in Nature (1988) 332:323.

The present invention also provides a new use of fragments and derivatives, in particular complementarity determining regions ("CDR's") of the above monoclonal anti-factor VIII antibodies as well as homologues thereof. For instance, antigen-binding fragments Fab, Fab' and F(ab')₂ generated by proteolytic digestion of the said monoclonal antibodies are provided, using methods well known in the art such as described by Stanworth et al., Handbook of Experimental Immunology (1978), vol.1 chapter 8 (Blackwell Scientific Publications). Such fragments, which contain the antibody binding site, have lost a number of properties of the parent antibody, such as complement activation or capacity to bind to Fc gamma receptors. Also included are single chain fragment variables (scFv), single variable domain fragments of the antibodies and combination of these fragments and of the above mentioned fragments.

The invention also provides a new use of soluble or membrane-anchored single-chain variable parts of the above monoclonal antibodies and a method for their obtention as follows. The DNA sequences of the variable parts of human heavy and light chains are amplified in separated reactions and cloned. A fifteen amino-acid linker sequence, for instance (Gly4 Ser)3, is inserted between VH and VL by a two-steps polymerase chain reaction (PCR), for instance according to Dieffenbach and Dveksler, "PCR Primer, a laboratory manual" (1995), Cold Spring Harbour Press, Plainview, NY, USA. The resulting fragment is then inserted into a suitable vector for expression of single chain fragment variable (scFv) as soluble or phage-displayed polypeptide. This can be achieved by methods well known to those skilled in the art, such as described by Gilliland et al., Tissue Antigens (1996) 47:1-20. The present invention also includes uses of a ligand comprising peptides representative of hypervariable regions of a monoclonal antibody which can be obtained by synthesis using an applied biosystem synthesizer, for instance a polypeptide synthesizer such as model 9050 available from Milligen (USA) or a model from a related technology, which alone or in combination with other or similar hypervariable regions will exert properties similar to that of the parent antibody.

Herein described is a pharmaceutical composition for the prevention or treatment of systemic inflammatory response syndrome associated to infection and/or sepsis and/or septic shock and/or septicemia in mammals, comprising as an active ingredient a ligand, preferably other than a polyclonal antibody, more preferably a human monoclonal antibody such as disclosed hereinabove, in admixture with one or more pharmaceutically acceptable carriers. Most preferably the said monoclonal antibody is a human monoclonal antibody, or a fragment or a homologue thereof, obtainable from the cell line KRIX 1 deposited with the Belgian Co-ordinated Collections of Micro-organisms under accession number LMBP 5089CB. The degree of homology with the said monoclonal antibody is preferably at least 80%, more preferably 90% and most preferably 95%, and the homology is preferably particularly in respect to the complementarity determining regions of the antibody. A ligand as used in accordance with the present invention may also include a synthetic polypeptide of equivalent potency. The pharmaceutical composition should comprise a therapeutically effective amount of the said above active ingredient, such as indicated hereinafter in respect to the method of treatment or prevention.

The pharmaceutical composition may further comprise, namely in view of a so-called combined treatment, a therapeutically effective amount of one or more anti-thrombotic agents. Such agents, as well as their usual dosage depending on the class to which they belong, are well known to those skilled in the art. Among numerous examples of such agents which may be included in the pharmaceutical compositions, the following non-limiting list may be particularly cited: heparin and low molecular weight heparin, anti-thrombin, heparinoids (such as danaparoid or dermatan sulphate), aprotinin-derived analogues, thrombomodulin, APC, defibrotide, TFPI, monoclonal antibodies against protease-activated receptors (PAR-1, PAR-3) and the like.

Suitable pharmaceutical carriers for use in pharmaceutical compositions described herein are described for instance in Remington's Pharmaceutical Sciences 16th ed. (1980) and their formulation is well known to those skilled in the art. They include any and all solvents, dispersion media, coatings, antibacterial and antifungal agents (for example phenol, sorbic acid, chlorobutanol), isotonic agents (such as sugars or sodium chloride) and the like. Additional ingredients may be included in order to control the duration of action of the monoclonal antibody active ingredient in the composition. Control release compositions may thus be achieved by selecting appropriate polymer carriers such as for example polyesters, polyamino acids, polyvinyl pyrrolidone, ethylenevinyl acetate copolymers, methylcellulose, carboxymethylcellulose, protamine sulfate and the like. The rate of drug release and duration of action may also be controlled by incorporating the monoclonal antibody active ingredient into particles, e.g. microcapsules, of a polymeric substance such as hydrogels, polylactic acid, hydroxymethylcellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition comprising the active ingredient may require protective coatings. The pharmaceutical form suitable for injection use include sterile aqueous solutions or dispersions and sterile powders for the extemporaneous preparation thereof. Typical carriers therefore include biocompatible aqueous buffers, ethanol, glycerol, propylene glycol, polyethylene glycol and mixtures thereof.

The present invention also provides the use of a partial inhibitor of factor VIII such as a monoclonal antibody (as disclosed herein-above) for the manufacture of a medicament or medicine for preventing and/or treating systemic inflammatory response syndrome associated to infection and/or sepsis and/or septic shock and/or septicemia in mammals, preferably in humans. The said partial inhibitor of factor VIII may be provided to the mammal in need of such prevention and/or treatment by any means well known to the person skilled in the art, i.e. preferably subcutaneously, intramuscularly, intradermally, intravenously, intra-arterially, parenterally or by catheterization. According to the present invention, the partial inhibitor of factor VIII may also be used for the manufacture of a medicament in conjunction or association with another medicament, for instance an anti-thrombotic agent such as disclosed hereinabove under the heading of pharmaceutical compositions.

Herein described is a method of treatment and/or prevention of blood circulation disorders following a systemic infection with gram (-) microorganisms in a mammal, preferably a human, comprising administering to a mammal in need of such treatment or prevention a therapeutically effective amount of a partial inhibitor of factor VIII, other than a polyclonal antibody, such as a monoclonal antibody as disclosed herein-above. Preferably the said partial inhibitor of factor VIII is a human or humanized monoclonal antibody obtainable from cell line KRIX 1 deposited with the Belgian Co-ordinated Collections of Micro-organisms under accession number LMBP 5089CB or an antigen-binding fragment Fab, Fab' or F(ab')₂, a complementarity determining region (CDR), a soluble or membrane-anchored single-chain variable fragment (scFv), a single variable domain or a derivative or combination of any of these elements.

A therapeutically effective amount as used herein means from about 1 microgram to about 5 milligrams per kilogram of body weight, more preferably from about 10 micrograms to about 1 milligram per kilogram of body weight of the mammal to be treated. More generally, the therapeutically effective amount may herein be defined as an anti-thrombin and/or activated protein C and/or tissue factor pathway inhibitor plasma level restoring amount. Such plasma levels may be easily and directly measured by the person skilled in the art by using methods such as disclosed in Laboratory Haematology (1989), ed. Chanarin, Churchill Livingstone, and in Laboratory techniques in Thrombosis, a manual (2nd revised edition of ECAT assay procedures, Eds Jespersen et al., Kluwer Academic Publishers (1999). It will be appreciated that, in view of the long half-life time of most IgG human antibodies, the partial inhibitors used in the present invention being monoclonal antibodies of the said class will provide, in a majority of cases, an efficient prevention and/or treatment with a single administration.

As preferred embodiments of the said disorders to be prevented or treated may be cited: systemic inflammatory response syndrome (SIRS) associated to infection, sepsis, septic shock, and septicemia. Preferably, the patient is provided with a bolus (intravenously injected) at a dosage determined by the ordinary skilled physician depending on criteria establishing the particular patient's clinical condition.

Methods of treatment and/or prevention described herein may include further treatment or prevention of the same pathologic condition by administering, preferably by sequentially administering, to the patient a therapeutically effective amount of one or more anti-thrombotic agents such as disclosed hereinabove under the heading of pharmaceutical compositions. Sequentially, as used herein, means that the partial inhibitor of thrombin formation, such as the partial inhibitor of factor VIII used in the present invention, and the known anti-thrombotic agent are administered to the patient sequentially but not simultaneously. More preferably, the partial inhibitor of factor VIII used in the present invention is administered in the first place and the anti-thrombotic agent is administered afterwards.

The partial inhibitors of factor VIII used in the present invention show the following advantages:
- they efficiently but only partially inhibit the co-factor activity of factor VIII, even when used in a more than 100-fold excess, i.e. they achieve a plateau effect in the inactivation of factor VIII, thereby avoiding the risk of overdosing the patient.
- human IgG antibodies exhibit a prolonged half-life time of three weeks (except for IgG3 which is one week), thus providing very stable plasma levels of the therapeutically active agent and allowing for a drastic reduction in the frequence of administration. Further, the use of human antibodies, fragments or homologues thereof, carries a minimal risk of inducing an immune response in the patient.
- they inhibit the function of FVIII to an extent sufficient to reduce, or partially inhibit, the formation of thrombin. Reduction, but not complete suppression, of the formation of thrombin prevents the development of disseminated intravascular coagulation (DIC) while allowing normal clot formation. Preventing DIC maintains normal organ perfusion and avoids organ dysfunction and failure.
- keeping the formation of thrombin under control reduces activation of the compensatory anti-inflammatory response. Thus, activated protein C is generated by direct thrombin cleavage and the effect of tissue factor pathway inhibitor is dependent on the presence of activated factor X, the activation of which is directly dependent on factor VIII co-factor activity. Limited depletion of circulating anti-thrombin (AT), which directly combines with thrombin, also occurs. In other words, both the pro-inflammatory and anti-inflammatory compensatory responses are maintained under control by regulating the rate of formation of thrombin.

Moreover, the maintenance of significant levels of physiological anti-thrombotic factors such as AT offers the possiblity of a combined therapy with the partial inhibitors of factor VIII used in the present invention. For instance, heparin exerts its major activity as anti-thrombotic agent by combining to AT and dramatically increases the capacity of the latter to inhibit thrombin. Therefore, heparin administration together with the therapeutically active compound used in this invention helps in delicately tuning the patient coagulation state.

The persistance of significant anti-factor VIII antibody levels in circulation after the acute phase of the SIRS should not deter from its use. Since the antibodies used in this invention only partially neutralize factor VIII, there is no inherent risk of spontaneous bleeding. To the contrary, persistant stable anti-thrombotic prevention is regarded as beneficial in patients kept under partial immobilization.

In most cases, a single administration of the partial inhibitor of factor VIII should be sufficient in view of the half-life of such human antibodies. However, under particular circumstances associated with the hypercatabolic state of sepsis, the administration may have to be repeated. In such cases, the absence of risk of overdosing (as explained hereinabove) precludes the need for monitoring factor VIII or antibody levels during treatment.

A further advantage of this invention is the lack of immunogenicity when the therapeutically active compound being used is of fully human origin. However, because in the treatment of SIRS only one or a limited number of administrations should be sufficient, the development of an immune response to an exogeneous partial inhibitor of factor VIII such as an antibody is unlikely. The invention therefore extends to the use of anti-factor VIII monoclonal antibodies of heterogeneous origin, such as antibodies or murine origin, and to the use of partially humanized antibodies, provided that such antibodies exhibit the same characteristics of only partial inactivation of factor VIII.

The present invention is further described by the following examples which are provided without limiting intention and only for illustrative purposes.

### EXAMPLE 1 - production of monoclonal antibodies derived from hemophilia A patients.

Human monoclonal antibodies of the desired specificity and characteristics are produced by transformation of B lymphocytes obtained from the peripheral blood of patients suffering from hemophilia A or acquired hemophilia. In order to elicit a more specific immunological response, patients are sought who have an impaired function of a physiologically active protein, e.g. factor VIII. By "impaired" is meant that some residual function is available but that this is less than is known for the wild-type of the same protein. A comparison between the self-protein and the wild-type protein should exhibit a difference in the two proteins, preferably in a region or domain which is of interest. The difference may be a deletion or a substitution of one or more amino acids with others. The patients are then administered enough of the wild-type protein to elicit an immunological response. Then, B-lymphocytes are extracted from the patients and selected based on the production of antibodies which have desirable properties. Although reference is made to "patients" above, the described methods may be applied generally to mammals. The above procedure results in a greater chance of obtaining antibodies which target the domain containing the defect.

B cells are transformed by infection with the Epstein-Barr virus and activation of surface antigens using techniques well known by those skilled in the art. Cell supernatants containing appropriate antibodies are identified by a specific assay procedure such as described in more details herein below.

Thus, antibodies towards factor VIII are identified by reacting the supernatant with polystyrene microtitration plates coated with factor VIII or with factor VIII/von Willebrand factor complexes. The binding of specific antibodies is detected by addition of a non human IgG reagent coupled to an enzyme. Addition of an enzyme substrate which is converted to a colored compound in the presence of the enzyme allows the detection of specific antibodies. Such methods referred to as enzyme-linked immunoassays (ELISA) are well known to those skilled in the art and described in details e.g. in Current Protocols in Immunology, chapter 2, John Wiley & Sons (1994).

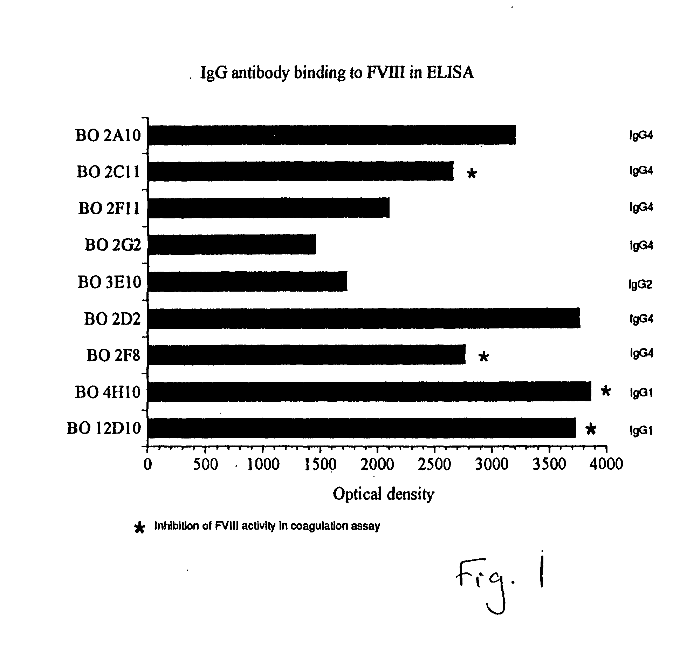

More specifically in the present case, the binding of anti-factor VIII IgG antibodies was detected by addition of a horseradish peroxidase labeled mouse monoclonal antibody specific for human Fcγ. The IgG subclass of the anti-factor VIII antibody was detected in ELISA, as presented in figure 1. The inhibition of factor VIII functional activity was tested in a functional coagulation assay as follows. Equal volume of the cell culture supernatant and of a pool of normal plasma were incubated for two hours at 37°C and the residual factor VIII activity measured thereafter. Those antibodies which significantly inhibit factor VIII activity are shown with an asterisk in figure 1.

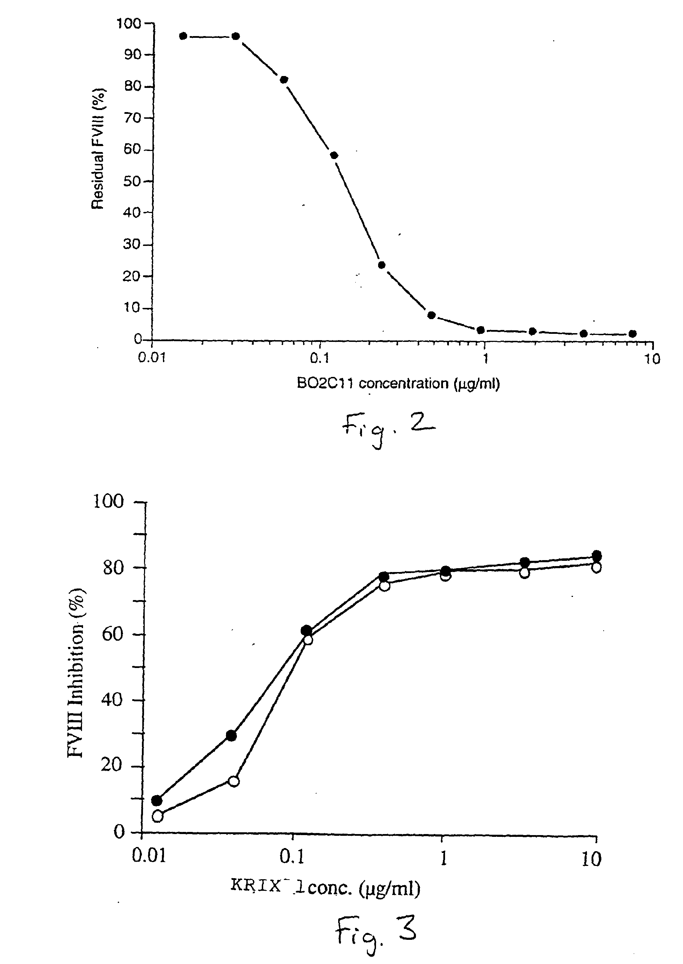

B cells (such as BO 2C11) producing anti-factor VIII antibodies are then expanded and cloned by limiting dilution as described for instance in *Current Protocols in Immunology* (see supra). Anti-factor VIII antibodies having the capacity to inhibit the pro-coagulant activity of factor VIII are identified using a chromogenic assay kit such as a factor VIII chromogenic assay commercially available either from Dade, Düdingen (Germany) or Coatest™ from Kabi Vitrum, Brussels (Belgium) or Chromogenix AB, Mölndal (Sweden) (see figure 2). Equal volumes of monoclonal antibodies BO 2C11 and a pool of normal blood plasma were incubated for 2 hours at 37°C. BO 2C11 concentrations before mixing are shown on the X axis. The reduction of factor VIII activity was measured in a coagulation assay and was expressed as a percentage of the activity obtained in the absence of antibody. The residual activity goes to zero asymptotically (complete inhibition).

Antibodies which inhibit factor VIII function with sufficient affinity but do not inhibit factor VIII pro-coagulant activity completely, even when used in large antibody excess, were selected. A representative example of such an antibody is provided in figure 3 where equal volumes of KRIX 1 and of recombinant factor VIII or of normal plasma being incubated for two hours at 37°C and concentrations (expressed in µg/ml) of KRIX 1 before mixing with plasma being as indicated, the residual factor VIII activity was measured using the above-mentioned chromogenic assay. Figure 3 interestingly shows about 60 % factor VIII inhibition at a concentration of 0.1 µg/ml and more interestingly an asymptotic factor VIII inhibition of about 80% in the whole range of concentrations from 0.5 to 10 µg/ml.

The thus selected antibodies are then produced in bulk culture and purified by affinity chromatography using methods well known to those skilled in the art.

The details of a non-limiting preparation technique are as follows. Human recombinant factor VIII (specific activity: 4000 IU/mg) was obtained from Hyland (Glendale, California); plasma-derived fVIII-vWf complex purified by ion exchange chromatography (specific activity ± 160 IU/mg protein; 15:1 vWf to fVIII weight ratio), and purified fVIII-depleted vWf (vWf to fVIII weight ratio 4800:1) were obtained from the Belgian Red Cross (Brussels, Belgium).

Peripheral blood samples were collected from donors suffering mild hemophilia and with inhibitors. The peripheral blood mononuclear cells (PBMC) were immortalized by Epstein-Barr virus infection concomitantly to the activation of surface antigens. 480 cell lines were screened by ELISA for production of antibodies towards factor VIII. One cell line, named KRIX 1, was successfully cloned by limiting dilution. Clonality was verified by RT-PCR amplification of mRNA coding for the V regions of the antibody heavy and light chains: a single sequence was obtained from 10 clones of PCR products. Purified antibodies were obtained by passage of KRIX 1 cell culture supernatant onto Protein-A Sepharose. An ELISA performed with IgG subclass- and light chain-specific antibodies identified KRIX-1 as an IgG4k.

Human monoclonal antibodies were purified by adsorption on immobilized Protein A (hi-Trap Protein A available from Pharmacia, Uppsala, Sweden). Fab fragments of human monoclonal antibody were prepared by papain digestion. 1 mg of a selected antibody was diluted to 500 µg/ml in phosphate buffer (40 mmol/L KH₂PO₄, 60 mM Na₂HPO₄.2H2O, 0.15M NaCl) containing 50 mmol/L L-cystein (Sigma), 1 mmol/L EDTA (Merck) and 10 µg papain (Sigma). The mixture was incubated for 3 hours at 37°C with continuous agitation. The reaction was stopped by addition of iodoacetamide to a final concentration of 75 mmol/L for 30 minutes at room temperature. The digested antibody was dialysed against phosphate-buffered saline (140 mmol/L NaCl, 67 mmol/L KCI, 20 mmol/L Na₂HPO₄, 4.4 mmol/L KH₂PO₄, pH 7.4). The undigested IgG and Fc fragments were then eliminated by passage over protein A sepharose (Hi Trap Protein A from Pharmacia). The Fab fragment was further purified by gel filtration chromatography on a Superdex 200 (Pharmacia).

Conventional methods were used for the detection of anti-factor VIII IgG antibodies, the determination of IgG subclass, and the evaluation of inhibition of factor VIII binding to vWf. For analyzing the inhibition of binding of rfVIII to a selected antibody by Fab and native antibody, Maxisorb polystyrene plates (Nunc) were coated for 2 hours with 50 µl of the antibody diluted to 5 µg/ml in glycine-buffered saline (20 mmol/L glycine, 34 mmol/L NaCI, pH 9.2). After washing, 50 µl of biotin-labeled rfvIII diluted to 1 µg/ml in Tris-casein (10 mmol/L tris(hydroxymethyl)-aminoethane, pH 7.3, containing 150 mmol/L NaCl and 0.5% casein) were mixed for 1 hour at 37°C with 50 µl of human IgG at various dilutions. A 50 µl aliquot of the mixture was added to the plates for 2 hours at room temperature. After washing, the binding of biotinylated rfVIII was detected by sequential addition of avidin-peroxydase and OPD.

rfVIII (final concentration 0.2 µg/mL) was incubated with human IgG antibody at different concentrations for 2 hours at 37°C and the residual factor VIII activity was assessed by a factor VIII chromogenic assay (e.g. Coatest^{™}, see *supra*). Inhibition of plasma factor VIII activity was measured by the Bethesda method, in which a pool of normal plasma collected in buffered trisodium citrate was used as factor VIII source. Residual factor VIII activity was assessed by a chromogenic or by a one-stage clotting assay.

### EXAMPLE 2 - Production of monoclonal antibodies by immunization in animals.

Alternatively, monoclonal antibodies having the same characteristics as disclosed in example 1 can be produced by on purpose immunization in animals. Thus, mice are injected with human factor VIII in Freund's adjuvant.

Monoclonal anti-human factor VIII antibodies are then obtained by fusion of spleen lymphocytes with a mouse myeloma cell line. Cell culture supernatants producing anti-factor VIII antibodies are identified and cloned by limiting dilution, using methods described in *Current Protocols in Immunology* (see supra). Further selection of inhibitors having the desired capacity to inhibit the pro-coagulant activity of factor VIII is carried out as described in example 1.

Monoclonal antibodies produced in mice are then humanized. Thus, sequences of the variable parts of mouse heavy and light chains are aligned with human immunoglobulin variable regions to identify human antibody with the greatest homology in framework regions. The DNA fragment encoding humanized variable regions are then synthesized by a PCR-based CDR (complementarity determining regions) grafting method as described for instance in Sato et al., Cancer Research (1993) 53:851-6. The final PCR product coding for the heavy chain variable part of the humanized antibody is digested and subcloned upstream of the human C gamma-1 gene in a first expression plasmid. The humanized light chain variable region of the final construction is inserted upstream of the C kappa gene in a second expression plasmid. The two constructions are then co-transfected into COS cells expression system.

### EXAMPLE 3 - Characterization of anti-factor VIII antibodies.

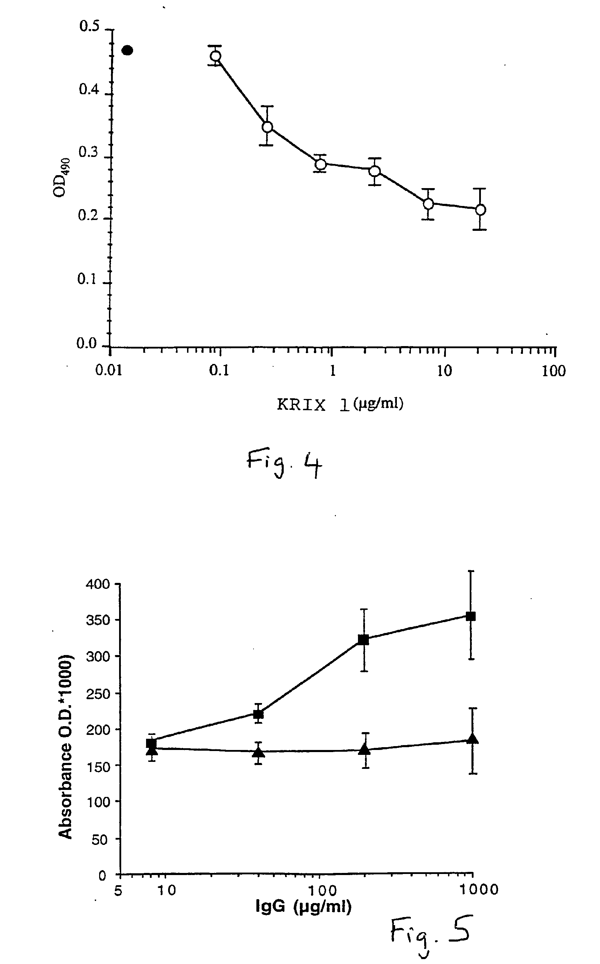

Monoclonal antibodies of either human (example 1) or animal (example 2) origin are characterized using an assay system by which their capacity to inhibit the binding of factor VIII to phospholipids is evaluated. Thus, polystyrene microtitration plates are coated with phosphatidyl-L-serine. Soluble recombinant factor VIII at 2 µg/ml final concentration is mixed for 30 minutes at 37°C with various concentrations of the antibody under evaluation. The mixture is then rapidly activated with thrombin and added to phosphatidyl-L-serine coated plates. The said plates were then incubated for two minutes at 21°C and the binding of factor VIII was detected by addition of the anti-factor VIII A1 domain mAbF14A2, for two minutes, followed by a two minute incubation with HRP-conjugated goat anti-mouse Fcγ. Results of this experiment are shown, for the monoclonal antibody produced from the cell line KRIX 1, in figure 4 wherein the average of activated factor VIII binding in the absence (closed symbols) or presence (open symbols) of the antibody, as well as the standard deviation of triplicates are indicated. Controls in the absence of factor VIII gave OD 490 lower than 0.05. Figure 4 thus clearly shows that the monoclonal antibody produced from cell line KRIX 1 inhibits significantly the binding of factor VIII to phospholipids but brings about incomplete inhibition even when added in large excess.

To demonstrate that in absence of plasma, KRIX 1 did not recognize the mutated factor VIII light chain of the donor, DNA fragments encoding wild-type and mutated factor VIII light chains were synthesized. The corresponding proteins were expressed in reticulocyte lysates. The correct folding of native and mutated light chains was determined by immunoprecipitation with the human monoclonal antibody BO2C11, which recognizes a conformational epitope within the carboxy-terminal part of the factor VIII light chain. Immunoprecipitation experiments indicated that BO2C11 bound wild-type and Arg2150His light chains, whereas KRIX 1 captured exclusively the wild-type light chain. Prolonged exposure of SDS-PAGE gels to the autoradiography film failed to detect any significant binding of KRIX 1 to the mutated light chain. Control experiments showed no binding to assay reagents other than factor VIII or factor VIII fragments, and preincubation with soluble rfVIII prevented the binding to methionine-labeled factor VIII fragments, confirming the binding specificity.

KRIX 1 did not recognize factor VIII in Western blotting indicating that the epitope recognized was conformational. Further epitope mapping was therefore performed with factor VIII fragments produced in reticulocyte lysates. Preliminary experiments had indicated that such an approach was efficient for the synthesis of factor VIII domains. The immunoprecipitation procedure using labeled factor VIII domains produced in reticulocyte lysate was validated by mapping the epitope recognized by the human monoclonal antibody BO2C11. A complete agreement was observed between the binding to factor VIII C2 deletion fragments produced in reticulocyte lysates and the binding to recombinant fragments produced in *E. coli* or COS cells. KRIX 1 bound to full-length light chain, to fragments corresponding to A3C1, C1C2 and the isolated C1 domain. In contrast, KRIX 1 did not bind to the C1 or C1C2 domains with the substitution Arg2150His, although in a control experiment, the Arg2150His C1C2 domain was bound by BO2C11 like its normal counterpart.

A search was made for other mutations in the light chain which could alter the binding of KRIX 1. As shown in Table 1 below, KRIX 1 inhibited the activity of all mutated factor VIII molecules tested so far, except those carrying the mutation Arg2150His.

**Table 1 - inhibition of FVIII activity in mild hemophilia A patients' plasma.**

| Factor VIII mutation | Factor VIII activity (IU/ml) | KRIX-1 Inhibition (%) |
|---|---|---|
| Arg1689Leu | 0.04 | >70 |
| Arg1749His | 0.39 | >70 |
| Gly1750Arg | 0.38 | >70 |
| Ala1824Val | 0.16 | >70 |
| Asp1825Gly | 0.17 | >70 |
| His1961Tyr | 0.24 | >70 |
| Arg1966Gln | 0.08 | >70 |
| Met2010Ile | 0.04 | >70 |
| Ser2011Asn | 0.23 | >70 |
| Val2016Ala | 0.05 | >70 |
| Asn2019ser | 0.13 | >70 |
| Leu2052Phe | 0.23 | >70 |
| Asp2074Gly | 0.13 | >70 |
| Factor VIII mutation | Factor VIII activity (IU/ml) | KRIX-1 Inhibition (%) |
| Thr2086Ile | 0.08 | >70 |
| Ile2098Ser | 0.20 | >70 |
| Phe2101 Leu | 0.06 | >70 |
| Asn2129Ser | 0.20 | >70 |
| Arg2150His | 0.03 | 0 |
| Pro2153Gln | 0.02 | 65 |
| Arg2159Leu | 0.16 | >70 |
| Trp2229Cys | 0.02 | >70 |
| Gln2246Arg | 0.08 | >70 |

In the use of KRIX 1 as a medicament to partially inhibit factor VIII, mild mutations of factor VIII will therefore not affect the effectiveness of the prophylactic or therapeutic treatment.

KRIX 1 inhibited the binding of factor VIII to vWf in a dose-dependent manner. The concentration of KRIX 1 required to achieve 50% inhibition (IC₅₀) of factor VIII binding was 0.25 µg/mL and more than 95% inhibition was obtained with 20 µg/mL of KRIX 1. Fab fragments of KRIX 1 also inhibited factor VIII binding to vWf. However, on a molar basis 15 times more Fab than native KRIX 1 was required to inhibit 50% of factor VIII binding to vWf. Additional experiments were performed in order to exclude that KRIX 1 Fab fragments could possibly still contain intact or partially digested antibody. SDS-PAGE analysis of the Fab fragments purified by protein A adsorption and gel filtration chromatography showed a single band. The presence of trace amounts of Fcγ fragments remaining bound to Fab fragments was excluded in ELISA. Wells with insoluble factor VIII were incubated with native or Fab KRIX 1. Binding of both Fab and native KRIX 1 could be detected by addition of peroxydase labeled anti-k light chain IgG. By contrast, addition of peroxydase labeled anti-Fcγ IgG did not reveal any specific binding even when 100 µg/ml of the Fab preparation were incubated into the wells. By comparison, the addition 0.1 µg/ml of the native antibody gave rise to a significant binding. In ELISA, a 15-fold higher concentration of Fab than of native antibody was required to inhibit 50% of the binding of biotinylated KRIX 1 onto insoluble factor VIII, indicating that the Fab KRIX 1 fragment had a lower affinity for factor VIII than the native antibody. Accordingly, the requirement for higher concentrations of KRIX 1 Fab than of native antibody to inhibit factor VIII binding to vWf should be attributed to the reduced affinity of KRIX 1 Fab fragments for factor VIII.

In order to determine whether KRIX 1 was representative of the polyclonal antibodies from the donor, a competitive assay was used. The binding of biotinylated KRIX 1 to insoluble factor VIII was measured in presence of increasing concentrations of either KRIX 1, polyclonal IgG from the donor, or control polyclonal IgG. IgG from the donor dose-dependently inhibited KRIX 1 binding to factor VIII. Concentrations of KRIX 1 and IgG from the donor inhibiting 50% of biotinylated KRIX 1 on factor VIII were respectively 0.3 µg/ml and 170 µg/ml, whereas no inhibition was observed with the control IgG.

### EXAMPLE 4 - production of monoclonal antibodies derived from hemophilia A patients and which bind to factor VIII and von Willebrand factor complex.

Alternatively, antibodies which reduce the release rate of factor VIII from von Willebrand factor are identified as follows. Polystyrene microtitration plates are coated with a specific antibody to von Willebrand factor (vWF). A solution of biotinylated recombinant factor VIII (0.5 µg/mL) complexed to vWF (5 µg/mL) is mixed with various concentrations of IgG from a donor (Figure 5 solid squares), e.g. the same patient as described above (from which KRIX 1 was derived), MoAb4H1D7, or of IgG from a non-hemophiliac subject (Figure 5 solid triangles). IgG at the indicated concentrations was added to microtitration plates coated with a mouse antibody, MoAb4H1D7 against vWF for an incubation of 2 hours at room temperature. After washing, factor VIII was activated by thrombin during two minutes at 37°C. Factor VIII bound to vWF was detected by addition of avidine peroxydase. Controls included the detection of bound biotinylated factor VIII in the absence of thrombin digestion (OD450=460+47.7SD) and of biotinylated recombinant factor VIII after thrombin digestion in the absence of antibody (OD450=160+16.OSD).

Results of these experiments for polyclonal antibodies are shown in figure 5, wherein the average values as well as the standard deviation of triplicates are indicated. Figure 5 thus clearly shows that a significantly higher proportion of activated factor VIII remains bound to the plate in the presence of increasing concentrations of the antibody, i.e. it demonstrates a reduction of the dissociation of activated factor VIII from vWF in the presence of an inhibitor antibody recognizing factor VIII bound to vWF. Monoclonal antibodies have been obtained from these polyclonal antibodies in accordance with the methods described herein. These antibodies exhibit the "plateau" effect at molar excess as described above with respect to the KRIX 1 antibody, thus indicating that the present invention may be extended to the use monoclonal antibodies and fragments and derivatives thereof which bind to complexes.

### EXAMPLE 5 - monoclonal antibodies derived from hemophilia A patients partially inhibit thrombin formation in vitro.

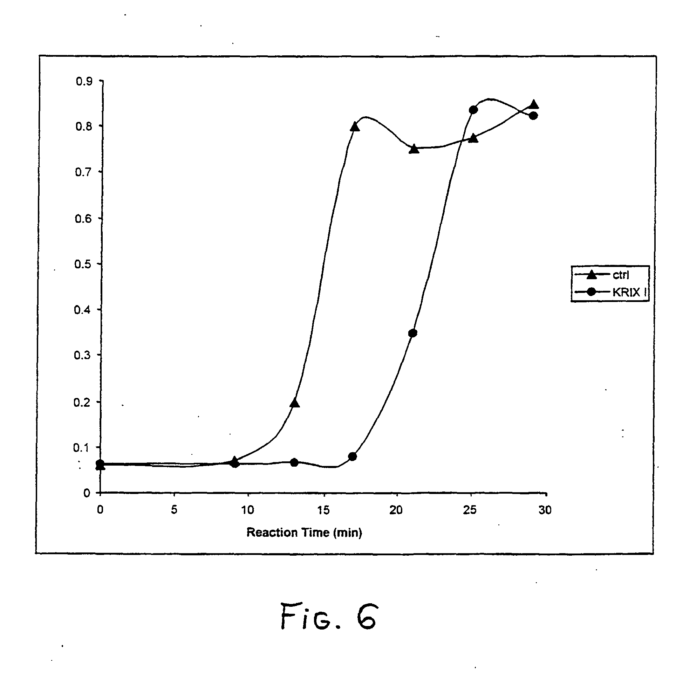

Human monoclonal antibodies produced according to example 1 were tested *in vitro* against the rate of thrombin formation using whole blood. Thus, citrated whole blood was recalcified and shaken at 900 rpm at 37°C in 24-well cell suspension culture plates avoiding contact activation. Samples were then transferred at regular intervals to a solution containing ethylenediaminetetraacetic acid (EDTA) in order to stop further coagulation activation. Following centrifugation, thrombin formation was then determined by a method well known in the art, e.g. by measuring optical density using the chromogenic substrate S-2238. Results of these tests are provided in figure 6, wherein absorption (expressed in nm) measured by the said method is indicated as a function of time for the sample incubated with KRIX 1 derived antibodies (noted KRIX 1) and for the control incubated without such antibodies (noted CTRL). This figure thus clearly shows that the addition of 2.5 µg/ml of human monoclonal antibodies derived from the cell line KRIX 1 significantly reduces (by about 1/3) but does not abolish the rate of thrombin formation as compared to the control.

### EXAMPLE 6 - in vivo reduction of thrombin formation by decreased levels of factor VIII.

Injection of endotoxin elicits the production of pro-inflammatory cytokines among which IL-6 and TNF-α are important for their interactions with the coagulation system. Thus, IL-6 increases the production of tissue factor and, consequently, the generation of thrombin. It also increases the production of fibrinogen by an independent mechanism. TNF-α increases the levels of plasminogen activation inhibitor type I (PAI-1) and thereby reduces fibrinolysis.

In order to show the effect of the presence of factor VIII on the latter reactions, groups of mice having the same genetic background (C57BI/6) with either normal factor VIII concentrations or the corresponding factor VIII knock-out strain were compared.

Three groups of six mice were constituted for each of the two strains. Mice were injected intraperitoneously with either 0.4 µg, 4 µg or 40 µg lipopolysaccharide (from E. coli serotype 0:111:B4) per 20 g of body weight. 90 minutes later, blood was taken by cardiac puncture in citrate buffer for evaluation of cytokine and coagulation factor levels. Plasma was obtained by centrifugation for 5 minutes at 5,000 rpm.

First, in order to determine whether comparable inflammatory changes had been inflicted to mice in the two strains, the level of IL-6 was evaluated. No interstrain differences were seen for each of the three different doses of lipopolysaccharide.

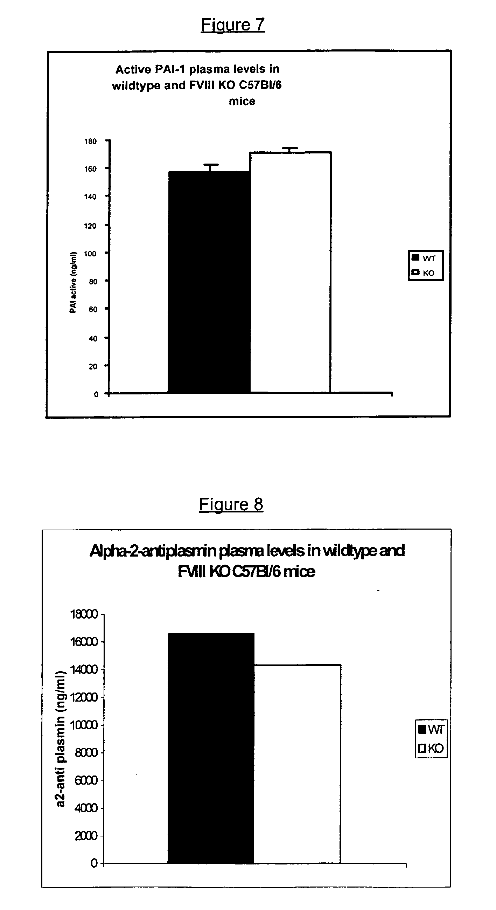

The extent to which the fibrinolytic pathway had been activated by a lipopolysaccharide injection of 40 µg per 20 g body weight was then evaluated by measuring concentrations of the two main pathway inactivators, namely PAI-1 and α₂-antiplasmin, using a sandwich-type ELISA with two specific monoclonal antibodies directed towards different sites of the molecule under evaluation. Figure 7 shows that the increase in PAI-1 concentration was similar in the two mouse strains, and figure 8 shows a similar behavior for α₂-antiplasmin. This indicates that the fibrinolytic activity was independent of the presence or the absence of factor VIII.

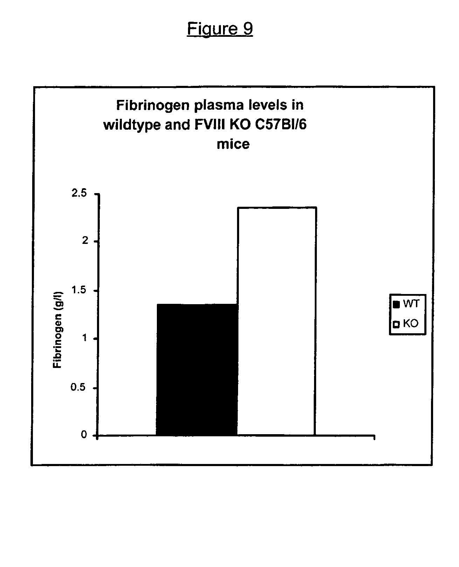

The evolution of fibrinogen plasma concentrations was used as a reading of its conversion into fibrin. As shown in Figure 9, the level of fibrinogen decreased by 45% in the group of mice with normal factor VIII level, as compared to the corresponding factor VIII knockout strain.

It is therefore concluded that, for an identical inflammatory trigger exemplified by increased IL-6 levels, and comparable activation of the fibrinolytic pathway, there was a significant reduction in fibrinogen degradation only in the presence of normal factor VIII concentrations. Lack or reduced levels of factor VIII therefore prevents the deposition of fibrin in the microvasculature, a key phenomenon in the systemic inflammatory syndrome leading to organ failure.

## Claims

1. Use of a partial inhibitor of factor VIII for the manufacture of a medicine for the prevention and/or treatment of systemic inflammatory response syndrome associated to infection and/or sepsis and/or septic shock and/or septicemia in mammals, wherein said partial inhibitor is a monoclonal antibody or a fragment thereof binding to a site of factor VIII or of a complex involving factor VIII, and having the capacity of inactivating factor VIII or a complex involving factor VIII by at least about 25 % and at most 95%, as determined by a chromogenic assay, when the said monoclonal antibody or fragment is in a molar excess with respect to factor VIII.

2. The use according to claim 1, wherein said partial inhibitor is a monoclonal antibody or fragment which recognizes an epitope located in the C1 domain of factor VIII.

3. The use according to claim 1 or 2, wherein said partial inhibitor is a human monoclonal antibody obtainable from the cell line named KRIX-1 deposited with the Belgian Coordinated Collections of Micro-organisms under accession number LMBP 5089CB, or an antibody having at least 80% sequence homology therewith within the CDR regions, or an antigen-binding fragment thereof.

4. The use according to any of claims 1 to 3, wherein said partial inhibitor is a monoclonal antibody having a reactivity identical to that of human monoclonal antibodies obtained from the cell line KRIX-1 deposited with the Belgian Coordinated Collections of Micro-organisms under accession number LMBP 5089CB, or an antigen-binding fragment thereof.

5. The use according to any of claims 1 to 4, wherein said fragment of said antibody is an Fab, Fab' or F(ab')₂ fragment, a soluble or membrane-anchored single-chain variable fragment, or a single variable domain or a combination thereof.

6. The use according to any of claims 1 to 5, wherein said medicine further comprises a therapeutically effective amount of an anti-thrombotic agent.

7. The use according to claim 6, wherein said anti-thrombotic agent is heparin.

## Patentansprüche

1. Verwendung eines Teil-Inhibitors von Faktor VIII für die Herstellung einer Medizin zur Vorbeugung und/oder Behandlung einer systemischen Entzündungsantwort (Systemic Inflammatory Response Syndrome), die mit Infektion und/oder Sepsis und/oder septischem Schock und/oder Septikämie bei Säugetieren in Zusammenhang steht, wobei der Teil-Inhibitor ein monoklonaler Antikörper oder ein Fragment davon ist, der bzw. das an eine Stelle von Faktor VIII oder eines Komplexes, der Faktor VIII beinhaltet, bindet und die Fähigkeit besitzt, Faktor VIII oder einen Komplex, der Faktor VIII beinhaltet, um mindestens etwa 25% und höchstens 95% zu inaktivieren, wie durch einen chromogenen Test bestimmt wird, wenn der monoklonale Antikörper oder das Fragment in Bezug auf Faktor VIII in einem molaren Überschuss vorhanden ist.

2. Verwendung nach Anspruch 1, wobei der Teil-Inhibitor ein monoklonaler Antikörper oder ein Fragment ist, der bzw. das ein Epitop erkennt, das sich in der C1-Domäne von Faktor VIII befindet.

3. Verwendung nach Anspruch 1 oder 2, wobei der Teil-Inhibitor ein humaner monoklonaler Antikörper ist, der von der Zelllinie mit der Bezeichnung KRIX-1 erhältlich ist, die bei Belgian Coordinated Collections of Micro-organisms unter der Zugangsnummer LMBP 5089CB hinterlegt ist, oder ein Antikörper mit mindestens 80% Sequenzhomologie mit diesem innerhalb der CDR-Regionen, oder ein Antigen-Bindungsfragment davon.

4. Verwendung nach einem der Ansprüche 1 bis 3, wobei der Teil-Inhibitor ein monoklonaler Antikörper mit einer Reaktivität ist, die mit jener von humanen monoklonalen Antikörpern identisch ist, die von der Zelllinie KRIX-1 erhalten werden, die bei Belgian Coordinated Collections of Micro-organisms unter der Zugangsnummer LMBP 5089CB hinterlegt ist, oder ein Antigen-Bindungsfragment davon.

5. Verwendung nach einem der Ansprüche 1 bis 4, wobei das Fragment des Antikörpers ein Fab-, Fab'- oder F(ab')₂-Fragment, ein lösliches oder membranverankertes einzelkettiges variables Fragment, oder eine einzelne variable Domäne oder eine Kombination davon ist.

6. Verwendung nach einem der Ansprüche 1 bis 5, wobei die Medizin des Weiteren eine therapeutische wirksame Menge eines antithrombotischen Mittels umfasst.

7. Verwendung nach Anspruch 6, wobei das antithrombotische Mittel Heparin ist.

## Revendications

1. Utilisation d'un inhibiteur partiel de facteur VIII pour la fabrication d'un médicament pour la prévention et/ou le traitement de syndrome de réponse inflammatoire systémique associé à une infection et/ou à une septicité et/ou à un choc septique et/ou à une septicémie chez les mammifères, dans laquelle ledit inhibiteur partiel est un anticorps monoclonal ou un fragment de ce dernier se liant à un site de facteur VIII ou d'un complexe impliquant le facteur VIII, et ayant la capacité d'inactiver le facteur VIII ou un complexe impliquant le facteur VIII d'au moins environ 25 % et d'au plus 95 %, comme on le détermine par un dosage chromogène, quand ledit anticorps monoclonal ou fragment est dans un excès molaire par rapport au facteur VIII.

2. Utilisation selon la revendication 1, dans laquelle ledit inhibiteur partiel est un anticorps monoclonal ou un fragment qui reconnaît un épitope situé dans le domaine C1 du facteur VIII.

3. Utilisation selon la revendication 1 ou 2, dans laquelle ledit inhibiteur partiel est un anticorps monoclonal humain que l'on peut obtenir à partir de la lignée cellulaire nommée KRIX-1 déposée avec les Collections Coordonnées Belges de Micro-organismes sous le numéro matricule LMBP 5089CB, ou un anticorps ayant une homologie de séquence d'au moins 80 % avec ce dernier à l'intérieur des régions CDR, ou un fragment de liaison d'antigène de ce dernier.

4. Utilisation selon l'une quelconque des revendications 1 à 3, dans laquelle ledit inhibiteur partiel est un anticorps monoclonal ayant une réactivité identique à celle d'anticorps monoclonaux humains obtenus à partir de la lignée cellulaire KRIX-1 déposée avec les Collections Coordonnées Belges de Micro-organismes sous le numéro matricule LMBP 5089CB, ou un fragment de liaison d'antigène de ce dernier.

5. Utilisation selon l'une quelconque des revendications 1 à 4, dans laquelle ledit fragment dudit anticorps est un fragment Fab, Fab' ou F(ab')₂, un fragment variable à chaîne simple soluble ou ancré dans la membrane, ou un domaine variable simple ou une combinaison de ces derniers.

6. Utilisation selon l'une quelconque des revendications 1 à 5, dans laquelle ledit médicament comprend en outre une quantité efficace d'un point de vue thérapeutique d'un agent antithrombotique.

7. Utilisation selon la revendication 6, dans laquelle ledit agent antithrombotique est de l'héparine.
